# EUROPEAN PATENT SPECIFICATION

(11) **EP 1 409 538 B1**
(45) Date of publication and mention of the grant of the patent: **07.01.2009**
(21) Application number: 02747753.8
(22) Date of filing: 22.07.2002
(51) Int. Cl.: C07K 14/495, C07K 14/65, C07K 16/04, C12N 9/08, A23J 1/20

(54) **PROCESS FOR OBTAINING GROWTH FACTOR (TGF-BETA AND IGF-1), LACTOPEROXIDASE AND IMMUNOGLOBULINS PREPARATIONS FROM MILK PRODUCTS HAVING LOW MUTUAL CROSS-CONTAMINATION**
VERFAHREN ZUR HERSTELLUNG VON PREPARATIONEN AUS MILCHPRODUKTEN, WELCHE WACHSTUMSFAKTOREN (TGF-BETA UND IGF-1), LACTOPEROXIDASE UND IMMUNGLOBULINE MIT NIEDRIGER GEGENSEITIGER VERUNREINIGUNG ENTHALTEN
PROCEDE D'OBTENTION DE FACTEUR DE CROISSANCE (TGF-BETA ET IGF-1), LACTOPEROXYDASE ET COMPOSITIONS D'IMMUNOGLOBULINES A PARTIR DE PRODUITS LAITIERS DOTES DE CONTAMINATION CROISEE MUTUELLE BASSE

(30) Priority: 20.07.2001 EP 01202794; 20.07.2001 EP 01202795
(43) Date of publication of application: 21.04.2004
(73) Proprietor: Campina B.V., 5301 LB Zaltbommel (NL); Numico Research B.V., 6700 CA Wageningen (NL)
(72) Inventor: KIVITS, Marinus, Gerardus, Cornelis, NL-5482 CC Schijndel (NL); GALAMA, Catharina, Maria, NL-5235 KL's Hertogenbosch (NL); HENDRIKS, Andor, Wilhelm, Joseph, NL 6035 PP Ospel (NL)
(74) Representative: van Westenbrugge, Andries
(86) International application number: PCT/NL2002/000496
(87) International publication number: WO 2003/008447

(56) References cited:
- EP-A- 0 335 554
- WO-A-01/25276
- WO-A-98/49272
- COX D A ET AL: "ISOLATION AND CHARACTERISATION OF MILK GROWTH FACTOR, A TRANSFORMING-GROWTH-FACTOR-BETA2-RELATED POLYPEPTIDE, FROM BOVINE MILK" EUROPEAN JOURNAL OF BIOCHEMISTRY, BERLIN, DE, vol. 197, 1 April 1991 (1991-04-01), pages 353-358, XP000612819 ISSN: 0014-2956 cited in the application
- ROGERS M-L ET AL: "TRANSFORMING GROWTH FACTOR BETA IN BOVINE MILK: CONCENTRATION, STABILITY AND MOLECULAR MASS FORMS" JOURNAL OF ENDOCRINOLOGY, BRISTOL, GB, vol. 151, no. 1, 1 October 1996 (1996-10-01), pages 77-86, XP000644717 ISSN: 0022-0795
- BELFORD D A ET AL: "PLATELET-DERIVED GROWTH FACTOR, INSULIN-LIKE GROWTH FACTORS, FIBROBLAST GROWTH FACTORS AND TRANSFORMING GROWTH FACTOR SS DO NOT ACCOUNT FOR THE CELL GROWTH ACTIVITY PRESENT IN BOVINE MILK" JOURNAL OF ENDOCRINOLOGY, BRISTOL, GB, vol. 154, no. 1, 1997, pages 45-55, XP000913489 ISSN: 0022-0795

## Description

### Technical field of the invention

The present invention relates to a process for obtaining fractions containing beneficial compounds from milk products (milk or whey). In particular according to the invention fractions are obtained enriched in growth factors compounds such as transforming growth factor β (TGF-β) or insulin like growth factor 1 (IGF-1).

### Background of the invention

It has been known for some time that milk products contain growth factors that can have a beneficial activity. These growth factors are present in very low concentrations in the milk product, which is why they are sometimes referred to as micronutrients. They can be characterised by their isoelectric point, which is relatively high compared to other milk proteins and their molecular weight. The present invention in particular concerns the growth factors TGF-β and IGF-1.

TGF-β is a multifunctional protein found in all mammalian tissues. Currently, five forms of TGF-β are known, β1 to β5. It has been implicated in the development, differentiation and growth of tissue and the control of immune system function and carcinogenesis. TGF-β can be isolated from natural sources (e.g. blood platelets), mammalian milk or colostrum or can be produced by recombinant cells.

IGF-1, an anabolic, i.e. growth promoting, growth factor, is a small protein (molecular weight about 7800) which plays an important role in bone metabolism. It has been shown to stimulate growth of cells in culture. Animal growth is also stimulated in pituitary deficient, normal and catabolic states. Kidney function is also improved. It can be produced using recombinant DNA technology, solid phase peptide synthesis, by isolating it from blood serum or from mammalian milk, e.g. bovine or human milk.

Extraction of such growth factors is known in the art. Hence, Eur. J. Biochem. 197, 353-358 (1991) describes that a TGF-β2 related polypeptide can be obtained from bovine milk. The method described is a combination of strong cation-exchange chromatography, low-pressure hydrophobic interaction chromatography, hydrophobic interaction HPLC, reversed phase HPLC and finally size exclusion HPLC steps. The disadvantage of this process is a multi-step process with a yield of less than 1 % TGF-β based on milk. According to this method only one fraction is isolated from milk. Thus, there is a need for a simplified process which produces more than one type of growth factors components like TGF-β and IGF-1 components but which can also produce other beneficial components.

One solution for this is given in WO 01/25276 of the applicant where it is described how fractions containing the growth factors TGF-β and IGF-1 can be extracted from a milk product via a passage through a HydroxyAPatite (HAP) column. Although the appropriate fractions of growth factors can be obtained, this process has some drawbacks. One of these drawbacks is that the life time of the HAP column and the yield per cycle are relatively low, which makes this process economically less feasible. Also, the HAP column binds lactoperoxidase making the process less efficient since the major part of the protein in this fraction consists of lactoperoxidase.

Rogers et al. "Transforming growth factor beta in bovine milk: concentration stability and molecular mass forms" J. Endocrinology 151 (1996) 77 - 86 describes the purification of TGF-beta from whey using a five-step chromatographic procedure successively involving cation exchange chromatography, two acid gel filtrations, and two filtrations using Deltapak C4 Column, and determination of the purified material by Edman degradation using an automated gas phase sequencer.

Accordingly, it is an object of the present invention to provide a process for obtaining fractions containing growth factors from milk, in particular for isolating TGF-β and IGF-1 from a milk product as relatively pure fractions (i.e. IGF-1 with a purity of more than 150 µg/g protein substantially free of TGF-β and TGF-β with a purity of more than 400 µg/g protein, preferably of at least 500 µg/g protein substantially free of IGF-1), thus providing a high content of growth factors by means of an economically feasible process. It is a further object of the invention to provide these growth factors in a form which is suitable for oral administration. It is a further object of the invention to recover TGF-β and IGF-1 from milk products as relatively pure fractions and simultaneously recover native lactoperoxidase (LP) in a high amount.

### Summary of the invention

According to the present invention, a process has been found to separate fractions rich in growth factors and other beneficial compounds, and at the same time produce a lactoperoxidase fraction with a high activity. The present invention thus provides a process for extracting fractions containing growth factor components from a milk product, comprising the steps of
a) recovering a basic fraction of the milk product by means of cationic exchange chromatography (CEC);
b) contacting a solution containing the fraction obtained in step a) with a hydrophobic interaction chromatography (HIC) resin comprising a carrier and a ligand attached to the carrier;
   wherein the ligand of the hydrophobic interaction chromatography resin is selected from an n-butyl group and a phenyl group;
c) eluting the hydrophobic interaction chromatography resin with an eluent to obtain a fraction containing growth factor compounds; and
   wherein the eluent of step c) contains substantially no alcohol.

### Detailed description of the invention

The present invention is concerned with the aforementioned process for extracting fractions containing growth factor components from a milk product.

The milk product which is used as a starting material for the present invention can be any mammalian milk or a milk derivative that contains growth factors, such as cheese whey or casein whey. Preferably bovine milk or milk derivative is used An advantage of using a milk product as a starting material for the invention is that, beside the desired beneficial compounds, in particular growth factors, also other compounds will be present in the eluted fractions, which can contribute to the effect of the desired compounds.

### Step a)-recovery by CEC

The cationic exchange resin used in step a) can be of any suitable type known in the field. It is preferred to use a cationic exchange resin of a mean particle size in excess of 100 µm or of a sufficient mechanical strength to resist high pressures. This has the advantage that the cationic exchange resin is resistant to high liquid loads, while the binding capacity is maintained. This makes it possible to process large amounts of liquid in short time, which is required for an industrially applicable process. Examples of suitable cationic exchange resins are S-Ceramic Hyper D, SP-Toyopearl, SP-Sepharose FastFlow and SP-Sepharose Big Beads.

Preferably the cationic exchange resin is equilibrated by buffering with a buffering agent of a pH value of 5.5 to 7.5, preferably 6.5. Suitable buffering agents for use herein in this process are those known in the art to the skilled man and typically selected from ammonium acetate, sodium phosphate and mixtures thereof, more preferably sodium phosphate. Then the milk product is passed through a column with the cationic exchange resin, for instance by pumping, whereby microcomponents adsorb from the starting material onto the cationic exchange resin. To prevent microbial growth, these processes are normally carried out at a temperature of 4 to 7 °C. However, it has been found that the viscosity at this temperature leads to an unacceptable pressure build-up. To overcome this problem, the Applicant has found that by preferably carrying out the adsorption at a temperature in the range of 15 to 30 °C, preferably 15 to 20 °C, lowered the viscosity of the milk or milk derivative, whilst still maintaining a relatively hygienic condition.

According to a preferred embodiment the starting material is pumped at a high surface velocity (more than 500 cm per hour) and at a high liquid load (100-600 bed volumes per hour) over a cationic exchange resin having a mean particle size of 100-300 µm, as described in US 5,596,082. According to this embodiment a process is realised which is highly favourable from an economic point of view, having outstanding industrial applicability.

After the adsorption step, it is preferred to rinse the cationic exchange resin column of any residual milk product (starting material) by washing with a salt solution buffered at a pH between 5.5 and 7.5, preferably 6.5 and having a salt concentration within the range of 0 to 0.20 molar, preferably within the range of 0.05 to 0.15 molar, more preferably of 0.10 molar.

Suitable salts for use herein are those commonly known by the man skilled in the art and are typically selected from sodium chloride, sodium sulphate, ammonium sulphate, potassium chloride, sodium phosphate, ammonium acetate, and mixtures thereof.

A preferred salt for the rinsing step of step a) is sodium chloride.

After adsorption of the desired components onto the ionic exchange resin, an elution step is carried out, preferably by eluting sequentially with eluents, preferably at least two eluents, of increasing salt concentration or pH so as to obtain a fraction with LP and growth factors and another with other beneficial components such as angiogenin and lactoferrin. Preferably the components are eluted with a salt solution buffered at a pH between 5.5 and 7.5, more preferably at a pH of about 6.5. Any of the salts above mentioned can be used herein for the elution of step a), however a preferred salt is sodium chloride or potassium chloride, more preferably sodium chloride. The salt concentration for the elution in step a) is within the range of from 0.15 to less than 1.5 M. Preferably, the first fraction is eluted with a salt solution concentration of from 0.15 to 0.5 M, preferably of from 0.20 to 0.40. This results in a fraction comprising LP, IgG, and angiogenine and the desired growth factors TGF-β, and IGF-1. The second fraction is then eluted with a salt solution concentration of from 0.50 to 1.5 M, preferably 0.9 to 1.1M. This results in a fraction comprising lactoferrin and optionally angiogenine.

### Step b)-contacting of the solution with HIC resin

According to the invention in step b) of the process a solution containing the fraction obtained in the cationic exchange chromatography step a) or preferably the growth factor containing fraction obtained in the cationic exchange chromatography step a) is passed over a hydrophobic interaction chromatography (HIC) resin comprising a carrier with a phenyl or butyl ligand attached to the carrier.

The carrier can be any carrier known in the art. For example a carrier based on cross-linked agarose, or a carrier based on a polymer of methacrylate/acrylate or polystyrenedivinylbenzene could be used. Examples of suitable resins are Toyopearl Phenyl 650M of Tosoh, Phenyl Sepharose Fast Flow (including the "High Sub" and "Low Sub variants), Phenyl Sepharose High Performance, Source Phenyl and n-butyl Sepharose 4 Fast Flow, all of Amersham Biosciences. A preferred resin for use in combination with a phenyl ligand is Phenyl Sepharose Fast Flow (including the "High Sub" and "Low Sub variants").

Preferred ligands for use herein are the phenyl group or n-butyl group, more preferably phenyl group. A most preferred ligand is phenyl.

The solution obtained from the ion exchange step and loaded onto the HIC resin contains the fraction obtained in step a), for instance in an amount of 0.1 to 10 %, in an aqueous solution of a salt, as herein before defined, and having a pH of 4 to 7. The salt concentration is of from 0.05 to 3 M. Where a phenyl or n-butyl ligand is used, the salt concentration is preferably of 0.25 to 3 M. A preferred salt for use herein is sodium chloride. This solution can further contain 0.01 to 0.2 M, preferably 0.01 to 0.1 M, more preferably 0.01 to 0.03 of a buffering agent as hereinbefore mentioned. Preferred buffering agents for use in this step are ammonium acetate or sodium phosphate.

The solution thus obtained is loaded onto the resin with a flow of about 5 to 30 bed volumes per hour, preferably 10 to 20 bed volumes per hour, whereby desired growth factors adsorb from the solution onto the HIC resin.

The HIC resin then preferably undergoes a wash step with a suitable wash liquid, which wash step encompasses pumping 2 to 5 bedvolumes of loading buffer (i.e. the salt/buffer solution without the fraction) over the column.

### Step c)-elution of the content of the HIC resin

After the adsorption step b) the HIC resin is eluted with a suitable eluent in step c).

By suitable eluent, it is meant an aqueous solution of 0.01 to 3.0 M salt with 0 to 50%(v/v) of a C1-C4 alcohol. The pH of the solution is of from 4.0 to 7.0 , preferably of about 4.5 to 6.5, more preferably of from 4.5 to 5.5. Typically the salt for use in step c) is as hereinbefore described for step b), and preferably comprises a mixture of buffering salts such as phosphate buffer and elution salts such as sodium sulphate, and more preferably is selected from ammonium acetate, sodium phosphate, sodium sulphate, ammonium sulphate, sodium chloride, potassium chloride, and mixtures thereof.

When a phenyl or n-butyl ligand is used, it is preferred to use as eluent an aqueous solution of 0.02 to 2.0 M salt. A most preferred salt is then sodium phosphate or mixtures of sodium phosphate with ammonium sulphate, with sodium sulphate, or with sodium chloride. Advantageously, the applicant has now found that when a phenyl or n-butyl ligand was used, the presence of alcohol within the aqueous solution was less necessary and even no longer necessary whilst still ensuring good elution of the fractions. Accordingly, for the purpose of the invention, it is preferred that when a phenyl or n-butyl ligand is used, that the eluent of step c) contains substantially no alcohol. By "substantially no alcohol", it is meant that the eluent of step c) contains less than 15% (vol/vol) of alcohol, preferably less than 5%, and more preferably contains no alcohol. This finding is particularly surprising, especially in view of the prior art where the use of a substantial amount of alcohol is always indicated for elution with HIC resin, such as described for example in Eur. J. Biochem. 197, 353-358 (1991) hereinbefore described. Further, the carrying out of the elution process without alcohol has been found advantageous in various aspects: compared to alcohol containing eluents, it has been found that the membranes used in the work-up and concentration of the eluted fractions, did not swell so much, thus resulting in a higher permeate flux during ultrafiltration, a lower transmembrane pressure, and therefore less energy consumption and less fast membrane ageing. Indeed, for the latter, membranes are often sensitive and thus deteriorated in the presence of alcohol. The present invention process solves this problem by providing a simple and efficient process. Further, as the presence of alcohol can then be reduced or can even be no longer required, no high capital investments are then required regarding the safety of the process such as explosion-proof process equipment.

Still, for the purpose of the invention and better separation of the components, it is preferred when a phenyl or n-butyl ligand is used to elute sequentially the fraction containing growth factor obtained from the previous step with eluents, preferably at least two eluents, of stepwise or linearly decreasing concentration or pH so as to respectively obtain a fraction enriched with LP and IGF-1, and a fraction enriched with TGF-β and IGF-1. In this instance, the first eluent which is used in step c) has preferably a concentration within the range of 0.1 to 2.0M ad the subsequent or second eluent which is used in step c) is preferably made with a salt of a phosphate buffering agent, more preferably within a concentration of 0.01 to 1.0 M salt, more preferably of from 0.01 to 0.5 M.

A more preferred salt is ammonium acetate. For the purpose of the invention and better separation of the components, it is preferred when a butyl ligand is used to elute sequentially the fraction containing growth factor obtained from the previous step with eluents, preferably with at least three eluents, of stepwise or linearly increasing alcohol concentration or pH so as to respectively obtain a fraction with LP, a fraction enriched with IGF-1 and a fraction enriched with TGF-β and other beneficial components such as LP, IgG, milk folate binding protein, lactogenin, angiogenin and RNase. In particular a gradient of 0-50% eluent results mainly in lactoperoxidase, 40 to 60% eluent results in an IGF-1 enriched fraction, 50-100% eluent results in an TGF-β enriched fraction.

The eluent is passed over the resin with a flow of about 5 to 30 bed volumes per hour, preferably 10 to 20 bed volumes per hour, whereby desired growth factors desorb from the HIC resin into the eluent.

Where a phenyl or n-butyl ligand is used in the process of the invention, the unbound fraction obtained after elution of the HIC resin is enriched in LP while the other fraction is enriched in TGF-β and IGF-1. This fraction can then be separated further by using a hydroxyapatite column as described in the application WO 01/25276. However, contrary to WO 01/25276, where the fraction containing the lactoperoxidase (LP) obtained via HAP was very small, the first fractions obtained in the present invention by HIC are very rich in LP, i.e. at least 800 mg/gram protein whilst the subsequent fractions are then rich in growth factors and other beneficial components. Accordingly, much better use and separation of the HAP column than in WO 01/25276 is made.

Thus the growth factor fraction enriched with TGF-β and IGF-1 and obtained in step c) is passed through a hydroxyapatite column, for instance by pumping, whereby the desired growth factors adsorb from the starting material onto the hydroxyapatite. The adsorption is preferably carried out at a pH within the range of from 5 to 7.5, preferably of from 5.5 to 7.5 and a salt, preferably sodium phosphate, concentration of 5 to 200 mmole/l.

After the absorption step the hydroxyapatite column is eluted sequentially with suitable eluting liquids. Possible eluents are sodium phosphate, sodium chloride and potassium chloride solutions. For the different fractions to be obtained these eluents must have an increasing salt concentration. It is also possible to apply an increasing pH gradient. Other possible eluents are known to the person skilled in the art. It is preferred that the overall concentration range of the salt solutions used is between 0.01 to 1.0 M.

According to the invention, to obtain an IGF-1 enriched fraction, the column is typically eluted with a phosphate buffer having a pH of 5.5 to 7.5 and a phosphate concentration of 0.05 to 0.2 M, preferably a pH of 5.7 to 6.5 and a phosphate concentration of 0.1 to 0.2 M. To obtain a TGF-β enriched fraction the column is subsequently eluted with a phosphate buffer having a pH of 5.5 to 7.5 and a concentration of at least 0.2 M, preferably a pH of 5.7 to 6.5 and a concentration of at least 0.25 M.

Still, if desired, the fractions obtained according to the present invention can be separated for further purification into their respective components by means of known methods. Examples of separation methods that can be used are ionic exchange chromatography, hydrophobic interaction chromatography, size exclusion chromatography or hydroxyapatite chromatography.

The final products can be treated further by techniques known in the art, to remove salt therefrom and/or to concentrate them. For salt removal for instance ultrafiltration or gel filtration can be used. For concentrating, the fractions can be lyophilised or spraydried.

### Pre-treatment

Before use in the present process, the milk can be subjected to a pretreatment such as mild pasteurization, and/or defattted using a centrifuge or a microfiltration step. Preferably, the starting material is first subjected to a minimal heat treatment. This is advantageous because
1) in such a heat treatment a considerable proportion of the bacteria naturally occurring in milk are killed and
2) the denaturation of lactoperoxidase and other milk serum proteins is minimized.

A minimal heat treatment is understood to mean heating to 80 °C at the most, preferably within the range of from 72° - 80° C for not more than a few seconds.

Further, it is highly advantageous to strip the starting material of fat before subjecting it to the adsorption and elution steps. It has been found that after fat removal the column in which the cationic exchange resin is contained hardly becomes greased or clogged up during the step of adsorption to the cationic exchange resin. This prevents undue pressure build up in the column and unfavourable shortening of the adsorption cycles.

It is preferred to remove fat by microfiltration because this effects at the same time the reduction of the microbial contamination of the starting material. In this connection, microfiltration is understood to mean filtration with a filter having a pore size between 0.1 and 10 µm.

### Product

The present invention also relates to the different fractions of growth factors obtainable and also obtained with the present process. The invention thus also comprises a product containing a TGF-β rich fraction essentially free of IGF-1 and a product containing an IGF-1 rich fraction essentially free of TGF-β.

Typically, the product containing a TGF-β rich fraction essentially free of IGF-1 has a weight ratio TGF-β to IGF-1 that is greater than 5, preferably greater than 50. This product in particular contains more than 400 µg TGF-β per gram protein, preferably more than 1500 µg TGF-β per gram protein and less than 8 µg IGF-I per gram protein, as determined by ELISA (Enzyme Linked Immuno Sorbent Assay). Generally, these fraction will contain 3000 µg TGF-β per gram protein at the most.

Preferably, a product obtainable by the invention process is also herein provided and which contains at least 1400 µg TGF-β per gram protein, preferably more than 2000 µg TGF-β per gram protein, more preferably at least 2500 µg TGF-β per gram protein and less than 8 µg IGF-I per gram protein.

The invention further comprises a product containing an IGF-1 rich fraction essentially free of TGF-β, wherein the weight ratio IGF-1 to TGF-β is greater than 10, preferably greater than 100. This product in particular contains more than 150 µg IGF-1 per gram protein, and less than 30, preferably less than 10 µg TGF-β per gram protein. Typically, such a product contains 3500 µg IGF-1 per gram protein at the most.

Accordingly, a product obtainable by the invention process is also herein provided and which contains more than 150 µg IGF-1 per gram protein, preferably at least 160 µg IGF-1 per gram protein, more preferably at least 180 µg IGF-1 per gram protein, and less than 30, preferably less than 10 µg TGF-β per gram protein.

As described before, during the loading of the HIC column, the unbound fraction contains the majority of the lactoperoxidase and gives a product containing lactoperoxidase having at least 1200 Units per mg, as determined with the ABTS method, essentially according to Shindler et al. (1976), European Journal of Biochemistry 65, 325 - 331. Product containing lactoperoxidase obtained by the invention process will contain at least 800 mg lactoperoxidase/ gram protein, preferably at least 850 mg/g to 900 mg/g protein.

The IGF-and TGF-fractions further contain about 30 to 50 % immunoglobulins on protein. Their main function is to interact with harmful micro-organisms such as bacteria. This prevents the micro-organism from entering the blood circulation system. This situation in particular occurs when the intestinal mucosa of the patient has been damaged as a result of treatment with chemotherapy.

The immunoglobulins can be isolated from milk of mammals which have been hyperimmunised against certain pathogens or they can be isolated from normal bovine milk or whey. With the present process, using normal cow's milk as a starting material, a preparation is obtained rich in immunoglobulins, comprising IgG and IgA. 30 to 50 % of the protein fraction consists of immunoglobulins of the type IgG and IgA, i.e. amounting to 300 mg/g protein to 500 mg/g protein.

The TGF-β and IGF-1 fractions obtained according to the invention contain binding factors which are released upon acidification. Thus the latent and active forms of both growth factors may be determined by e.g performing a growth factor specific ELISA in the presence or absence of an acid treatment of the sample, respectively. The binding factors fulfil a role in the modulation of the growth factor activity and may protect the growth factors during passage through the gastrointestinal tract

The IGF-and TGF-fractions obtained according to the invention can be used in the treatment and/or prevention of malfunction or disease of the intestinal mucosa, e.g. as the result of chemotherapy or radiotherapy.

The present invention is further illustrated by means of the following non-limiting examples. In the examples the following methods were used to analyse the products obtained.

Test kits for the determination of TGF-β and IGF-1 are commercially available. Test kit used: Quantikine® for determination of human TGF-β from R&D Systems.

TGF-β is determined using a quantitative sandwich enzyme immunoassay technique (ELISA). A monoclonal antibody specific for human TGF-β2 has been pre-coated onto a microplate. Human and bovine TGF-β are identical so that the antibody will detect the bovine form. Standards and samples are pipetted into the wells and any TGF-β present is bound by the immobilized antibody. Prior to this step, since the TGF-β in milk is present in a latent form, it is first activated by an acid treatment to determine the total TGF-β concentration. This activation step is left out to determine the amount of free (unbound) TGF-β.

After washing away any unbound substances, an enzyme-linked polyclonal antibody specific for TGF-β2 is added to the wells. Following a wash to remove any unbound antibody-enzyme reagent, a substrate solution is added to the wells and colour develops in proportion to the amount of TGF-β2 bound in the initial step. The colour development is stopped and the intensity of the colour measured.
TGF-β in samples is expressed as µg/g protein.
IGF-1: test kit used: IGF-1 ELISA DSL-10-2800 from Diagnostic Systems Laboratories, Inc.
IGF-1 is also determined by an enzymatically amplified "two-step"sandwich-type immunoassay similar to TGF-β. Samples, controls and prediluted unknowns are incubated in microtitration wells which have been coated with anti-IGF-1 antibody. IGF-1 in milk can be bound to binding proteins, and therefore, an activation step using acid similar to TGF-β is used when determining total IGF-1 concentration. The amount of free IGF-1 is determined when the activation step is left out.
IGF-1 in samples is expressed as µg/g protein.

### Protein

Protein in samples is determined with the Bradford method using Lactoferrin to make the standard curve. Alternatively, protein can be measured using detection of the peptide bond at wavelengths of 214-220 nm.

Lactoperoxidase in samples is determined by the SDS PAGE electrophoresis (homogeneous gelconcentration 20%; 2% cross-linking).

Immunoglobulin IgG and angiogenine are determined by SDS electrophoresis and Western blotting (on nitrocellulose membrane).

Milk Folate Binding Protein, Lactogenin and RNAse are determined by SDS electrophoresis, followed by Western blotting on Sequi-blot PVDF membranes for protein sequencing by Alta Bioscience, University of Birmingham, UK.

The following are non-limiting examples illustrating the present invention:
In experiments where milk was used as starting material, the concentration of IGF-1, TGF-β and lactoperoxidase was as follows: IGF-1: 0.045 µg/g protein; TGF-β: 0.9 µg/g protein;
lactoperoxidase: 1.5 mg/g protein.

### Example 1: Isolation of IGF-1, TGF- β and LP from milk using Phenyl Sepharose Fast Flow low sub HIC resin.

An ion exchange chromatography (IEC) column having a diameter of 10cm was packed with 1L of a strong cation exchanger (SP Sepharose Big Beads, Amersham Biosciences).

The column was preconditioned using a phosphate buffer (pH 6.5 0.025 M phosphate). The fat fraction of the milk was removed by means of centrifugation and 360 1 of the resulting skim milk was passed over the column at room temperature at a flow rate of 100BVH (Bed Volumes per Hour). The column was washed with 5L of a 0.10M NaCl pH 6.5 solution. The adsorbed proteins were then fractionated by subsequently eluting the column with:
a) 5L of a 0.25M NaCL solution, pH 6.5
b) 5L of a 1.00M NaCl solution, pH 6.5.
   Fraction a) contained predominantly lactoperoxidase and was rich in IGF-1 and TGF-β. Fraction b) was rich in angiogenin and lactoferrin. According to the results, fraction a) contained 780 mg LP/gr. protein, 25µg IGF-1/gr. protein and 115µg TGF-β/ gr protein. To the eluted fraction a) was added ammoniumsulphate untill the concentration of the ammoniumsulphate in the solution was 1 M. The pH was adjusted to 5.0. The solution was subsequently loaded onto a column containing 0.75L Phenyl Sepharose Fast Flow low sub (Amersham Biosciences). The column was washed with 2 L 0.025 phosphate and 1.0 M ammoniumsulphate pH 5.0 The adsorbed proteins were then fractionated by subsequently eluting the column with:
c) 3 L 0.025M phosphate with 0.6 M ammoniumsulphate pH 5.0
d) 3 L 0.025M phosphate pH 5.0
   The unbound protein fraction and fraction c) contained 850 mg LP/gr. protein (specific activity 1200 units/mg) and 10 µg IGF-1/ gr. protein. Fraction d) contained 1000 µg TGF-β/ gr. protein, and 125 µg IGF-1/gr. protein.
   For a further purification step, the eluted fraction d) is loaded onto an column containing 0.2L Hydroxyapatite (BioRad ceramic HAP type I, 40 µm) to separate TGF-β from IGF-1. The column was washed with with a buffer containing 60 mM phosphate pH 6.0.
   The adsorbed proteins were then fractionated by subsequently eluting the column with:
e) 1 L 0.14M phosphate pH 6.0
f) 0.6 L 0.5M phosphate pH 7.0

Fraction e) contained 165 µg IGF-1/ gr. protein and < 1µg TGF-β/ g protein. The fraction further contained 30 - 50 % w/w immunoglobulines. The other major component identified was RNAse.

Fraction f) contained 2500 µg TGF-β /g protein and was low in IGF-1 (<5 µg IGF-1/g protein). The fraction further contained lactoperoxidase and IgG.

### Example 2: Isolation of IGF-1, TGF- β and LP from milk using Toyopearl phenyl HIC resin and different loading and eluting conditions.

To the fraction a) obtained by the IEC elution was added NaCl until a 3 M salt concentration was reached. The pH was adjusted to 5.0. With this solution a column was loaded containing 0.75 L Toyopearl Phenyl 650M (HIC resin from TosoHaas). The column was washed with 2 L 0.025M phosphate and 3M NaCl pH 5.0. The adsorbed proteins were then fractionated by eluting with a linear gradient of 37.5 L 0.025M phosphate buffer pH 5.0 of 3M to 0M NaCl. The unbound fraction, the wash fraction and the first part of the salt gradient contained predominantly lactoperoxidase. The growth factors IGF-1 and TGF- β eluted together over a large part of the gradient. This fraction contained 210 µg IGF-1/gr. protein and 875 µg TGF- β/gr. protein. For further separation of IGF-1 and TGF- β, the hydroxyapatite step (using BioRad ceramic HAP type I, 40 um) is used as described in example 1.

### Example 3: Isolation of IGF-1, TGF- β and LP from milk using Source Phenyl HIC resin and different loading and eluting conditions.

To the fraction a) obtained by the IEC elution was added NaCl until a 3 M salt concentration was reached. This solution was adjusted to pH 5.0. With this solution a column containing 0.75 L Source Phenyl (HIC resin, Amersham Biosciences) was loaded. The column was washed with 2 L 0.025M phosphate and 3M NaCl buffersolution pH 5.0. The adsorbed proteins were fractionated by subsequently eluting the column with:
g) 5 L 0.025M phosphate with 1.5M NaCl pH 5.0
h) 5 L 0.025M phosphate pH 5.0.

Fraction g) contained predominantly LP and fraction h) contained 200 µg IGF-1/gr. protein and 714 µg TGF-β/ gr. protein.

Further separation of IGF en TGF was achieved by applying the hydroxyapatite purification step as described in example 1.

### Example 4: Isolation of IGF-1, TGF- β and LP from milk using Phenyl Sepharose Fast Flow high sub HIC resin and different loading and eluting conditions.

To the fraction a) obtained by the IEC elution is added sodiumsulphate until a salt concentration is reached from 0.6M. The pH of the solution is kept on 6.5. With this solution a column containing 0.75 L Phenyl Sepharose Fast Flow high sub (HIC resin, Amersham Biosciences) is loaded. The column is washed with 2 L 0.025M phosphate and 0.6M sodium sulphate buffer solution pH 6.5. The adsorbed proteins were fractionated by subsequently eluting the column with:
i) 3 L 0.025M phosphate and 0.2M sodium sulphate pH 6.5
j) 3 L 0.025M phosphate pH 6.5

The unbound fraction and fraction i) contained predominantly lactoperoxidase and some IGF-1. The fraction j) contained 167 µg IGF-1/ gr. protein and 2000 µg TGF-β/ gr. protein.

Further separation of IGF-1 and TGF- β was achieved by applying the hydroxyapatite purification step as described in example 1.

### Example 5: Isolation of IGF-1, TGF- β and LP from milk using n-butyl Sepharose.

To the fraction a) obtained by the IEC elution was added NaCl until a 2 M salt concentration was reached. The pH was adjusted to 5.0. With this solution a column was loaded containing 0.75L n-Butyl Sepharose 4 Fast Flow (Amersham Biosciences). The column was washed with 3 L 0.025M phosphate and 1.5 M NaCl pH 5.0.

The adsorbed proteins were then eluted with 3 L 0.025M phosphate pH 5.0.(fraction k)) The unbound fraction and washfraction contained 850 mg LP per gram protein (specific activity 1200 units/mg).

The eluted fraction k) contained 725 µg TGF-β per gram protein and 158 µg IGF-1 per gram protein.

Further separation of IGF-1 and TGF-β was achieved by appling the hydroxyapatite purification step as described in example 1.

## Claims

1. Process for extracting fractions containing growth factors from a milk product, comprising the steps of
a) recovering a basic fraction of the milk product by means of cationic exchange chromatography;
b) contacting a solution containing the fraction obtained in step a) with a hydrophobic interaction chromatography resin comprising a carrier and a ligand attached to the carrier;
wherein the ligand of the hydrophobic interaction chromatography resin is selected from an n-butyl group and a phenyl group;
c) eluting the hydrophobic interaction chromatography resin with an eluent to obtain a fraction containing growth factor compounds; and
wherein the eluent of step c) contains substantially no alcohol.

2. Process according to claim 1, wherein the ligand is a phenyl group.

3. Process according to claim 1 or 2, wherein the solution in step b) comprises the fraction obtained in step a) in an aqueous solution of 0.05 to 3 M salt having a pH of 4 to 7.

4. Process according to claim 3, wherein the solution further contains 0.01 to 0.2 M of a buffering agent.

5. Process according to any one of the preceding claims, wherein the eluent used in step c) is an aqueous solution of 0.01 to 3.0 M salt having a pH of 4 to 7.

6. Process according to any one of the preceding claims, wherein in step c) the resin is eluted stepwise or linearly with decreasing concentrations of salt or pH.

7. Process according to any one of the preceding claims, wherein the fraction obtained in step c) is passed over a hydroxyapatite column and the hydroxyapatite column is eluted stepwise with a phosphate buffer having a pH of 5.5 to 7.5 and a phosphate concentration of 0.05 to 0.2 M and then a phosphate buffer having a pH of 5.5 to 7.5 and a phosphate concentration of at least 0.2 M.

8. Process according to any one of the preceding claims, wherein the eluent used in step c) is an aqueous solution of 0.02 to 2.0 M salt.

9. Process according to any of the preceding claims, wherein the milk product is any mammalian milk from which fat has been removed, or whey.

10. Process according to any one of the preceding claims, which extracted fraction contains more than 2000 µg TGF-β per gram protein and less than 8 µg IGF-1 per gram protein.

11. Process according to any one of the preceding claims, which extracted fraction according to claim 10 contains immunoglobulins in an amount of 300 mg/g protein to 500 mg/g protein.

12. Process according to any one of claims 1-9, which extracted fraction contains lactoperoxidase with an activity of at least 1200 Units/mg, and in an amount of from 800 - 900 mg/g protein.

## Patentansprüche

1. Verfahren zur Extraktion von Fraktionen, die Wachstumsfaktoren enthalten, aus einem Milchprodukt, das die folgenden Schritte umfasst:
a) Gewinnen einer Basisfraktion des Milchprodukts mittels Kationenaustauschchromatographie;
b) Inkontaktbringen einer Lösung, die die Fraktion enthält, die in Schritt a) erhalten wurde, mit einem hydrophoben Interaktionschromatographieharz, das einen Träger und einen Liganden umfasst, der an dem Träger haftet; wobei der Ligand des hydrophoben Interaktionschromatographieharzes ausgewählt ist aus einer n-Butylgruppe und einer Phenylgruppe;
c) Eluieren des hydrophoben Interaktionschromatographieharzes mit einem Elutionsmittel, um eine Fraktion zu erhalten, die Wachstumsfaktorverbindungen enthält; und
wobei das Elutionsmittel aus Schritt c) im Wesentlichen keinen Alkohol enthält.

2. Verfahren nach Anspruch 1, wobei der Ligand eine Phenylgruppe ist.

3. Verfahren nach Anspruch 1 oder 2, wobei die Lösung in Schritt b) die Fraktion, die in Schritt a) erhalten wurde, in einer wässrigen Lösung von 0,05 bis 3 M Salz umfasst, die einen pH-Wert von 4 bis 7 aufweist.

4. Verfahren nach Anspruch 3, wobei die Lösung ferner 0,01 bis 0,2 M einer Puffersubstanz enthält.

5. Verfahren nach einem der vorhergehenden Ansprüche, wobei das in Schritt c) verwendete Elutionsmittel eine wässrige Lösung von 0,01 bis 3,0 M Salz ist, die einen pH-Wert von 4 bis 7 aufweist.

6. Verfahren nach einem der vorhergehenden Ansprüche, wobei das Harz in Schritt c) schrittweise oder linear mit abnehmenden Salzkonzentrationen oder pH-Werten eluiert wird.

7. Verfahren nach einem der vorhergehenden Ansprüche, wobei die in Schritt c) erhaltene Fraktion über eine Hydroxyapatitsäule geleitet wird und die Hydroxyapatitsäule schrittweise mit einem Phosphatpuffer, der einen pH-Wert von 5,5 bis 7,5 und eine Phosphatkonzentration von 0,05 bis 0,2 M aufweist, und dann einem Phosphatpuffer, der einen pH-Wert von 5,5 bis 7,5 und eine Phosphatkonzentration von mindestens 0,2 M aufweist, eluiert wird.

8. Verfahren nach einem der vorhergehenden Ansprüche, wobei das in Schritt c) verwendete Elutionsmittel eine wässrige Lösung von 0,02 bis 2,0 M Salz ist.

9. Verfahren nach einem der vorhergehenden Ansprüche, wobei das Milchprodukt jede Säugetiermilch, aus der das Fett entfernt wurde, oder Molke ist.

10. Verfahren nach einem der vorhergehenden Ansprüche, dessen extrahierte Fraktion mehr als 2.000 µg TGF-β je Gramm Protein und weniger als 8 µg IGF-1 je Gramm Protein enthält.

11. Verfahren nach einem der vorhergehenden Ansprüche, dessen extrahierte Fraktion nach Anspruch 10 Immunoglobuline in einer Menge von 300 mg/g Protein bis 500 mg/g Protein enthält.

12. Verfahren nach einem der Ansprüche 1 bis 9, dessen extrahierte Fraktion Lactoperoxidase mit einer Aktivität von mindestens 1.200 Einheiten/mg, und in einer Menge von 800 bis 900 mg/g Protein enthält.

## Revendications

1. Processus d'extraction de fractions contenant des facteurs de croissance d'un produit laitier, comprenant les étapes consistant à
a) récupérer une fraction basique du produit laitier au moyen d'une chromatographie par échange de cations ;
b) mettre en contact une solution contenant la fraction obtenue à l'étape a) avec une résine de chromatographie hydrophobe comprenant un support et un ligand attaché au support ;
dans lequel le ligand de la résine de chromatographie hydrophobe est sélectionné à partir d'un groupe n-butyle et un groupe phényle ;
c) éluer la résine de chromatographie hydrophobe avec un éluant pour obtenir une fraction contenant des composés de facteur de croissance ; et
dans lequel l'éluant de l'étape c) ne contient essentiellement pas d'alcool.

2. Processus selon la revendication 1, dans lequel le ligand est un groupe phényle.

3. Processus selon la revendication 1 ou la revendication 2, dans lequel la solution de l'étape b) comprend la fraction obtenue à l'étape a) dans une solution aqueuse de 0,05 à 3 M de sel ayant un pH de 4 à 7.

4. Processus selon la revendication 3, dans lequel la solution contient en outre de 0,01 à 0,2 M d'un tampon.

5. Processus selon l'une quelconque des revendications précédentes, dans lequel l'éluant utilisé à l'étape c) est une solution aqueuse de 0,01 à 3,0 M de sel ayant un pH de 4 à 7.

6. Processus selon l'une quelconque des revendications précédentes, dans lequel à l'étape c) la résine est éluée par étape ou de manière linéaire avec des concentrations dégressives de sel ou du pH.

7. Processus selon l'une quelconque des revendications précédentes, dans lequel la fraction obtenue à l'étape c) est passée sur une colonne d'adsorption sur gel d'hydroxyapatite et la colonne d'adsorption sur gel d'hydroxyapatite est éluée par étape avec un tampon phosphate ayant un pH de 5,5 à 7,5 et une concentration phosphatique de 0,05 à 0,2 M, puis un tampon phosphate ayant un pH de 5,5 à 7,5 et une concentration phosphatique d'au moins 0,2 M.

8. Processus selon l'une quelconque des revendications précédentes, dans lequel l'éluant utilisé à l'étape c) est une solution aqueuse de 0,02 à 2,0 M de sel.

9. Processus selon l'une quelconque des revendications précédentes, dans lequel le produit laitier est un lait de mammifère quelconque duquel la matière grasse a été éliminée, ou du lactosérum.

10. Processus selon l'une quelconque des revendications précédentes, laquelle fraction extraite contient plus de 2000 µg de TGF-β par gramme de protéine et moins de 8 µg d'IGF-1 par gramme de protéine.

11. Processus selon l'une quelconque des revendications précédentes, laquelle fraction extraite selon la revendication 10 contient des immunoglobulines dans une quantité de 300 mg/g de protéine à 500 mg/g de protéine.

12. Processus selon l'une quelconque des revendications 1 à 9, laquelle fraction extraite contient de la lactoperoxydase avec une activité d'au moins 1200 unités/mg, dans une quantité de 800 - 9000 mg/g de protéine.
